# EUROPÄISCHE PATENTSCHRIFT

(11) **EP 0 633 789 B1**
(45) Veröffentlichungstag und Bekanntmachung des Hinweises auf die Patenterteilung: **25.11.1998**
(21) Anmeldenummer: 93906611.4
(22) Anmeldetag: 25.03.1993
(51) Int. Cl.: A61L 15/26, A61L 15/28

(54) **WUNDVERBAND, WUNDAUFLAGE ODER TRÄGERMATRIX**
WOUND DRESSING, WOUND PLASTER OR CARRIER MATRIX
BANDE DE PANSEMENT, PANSEMENT OU MATRICE DE SUPPORT

(30) Priorität: 02.04.1992 DE 4210891; 12.12.1992 DE 4242015
(43) Veröffentlichungstag der Anmeldung: 18.01.1995
(73) Patentinhaber: RADEMACHER, Karl-Heinz, 30826 Garbsen (DE)
(72) Erfinder: RADEMACHER, Karl-Heinz, 30826 Garbsen (DE)
(74) Vertreter: Werner, Hans-Karsten, Dr.Dipl.-Chem.
(86) Internationale Anmeldenummer: EP9300723
(87) Internationale Veröffentlichungsnummer: WO9319789

(56) Entgegenhaltungen:
- EP-A- 0 190 814
- EP-A- 0 335 669
- WO-A-92/03172
- BIOMATERIALS Bd. 7, Nr. 1, Januar 1986, GUILDFORD,SURREY, GB Seiten 67 - 72 B. KICKH FEN 'CHEMICAL AND PHYSICAL PROPERTIES OF A HYDROGEL WOUND DRESSING'

## Beschreibung

Gegenstand der vorliegenden Erfindung sind Wundverbände, Wundauflagen oder Trägermatrices, bestehend aus einem bioverträglichen, offenporigen Polyurethanschaum und einem eingelagerten Hydrogel aus Guar gum sowie Tensiden und/oder weiteren Zusatzstoffen sowie Verfahren zur Herstellung derselben, wobei das Guar gum sowie die Tenside und/oder weiteren Zusatzstoffe in situ miteingeschäumt wurden bei der Umsetzung von Wasser mit hydrophilen Polyurethan-Prepolymeren auf Basis von Toluoldiisocyanat (TDI) und/oder Methylendiphenyldiisocyanat (MDI).

Aus der WO 92/03172 der Anmelderin ist ein Wundauflagensystem bekannt, welches unter anderem auch aus einem bioverträglichen offenporigen Polyurethanschaum und einem eingelagerten Hydrogel aus Guar gum sowie gegebenenfalls weiteren Zusatzstoffen besteht. Dabei wird, ausgehend von einem vorgefertigten Polyurethanschaum, eine Lösung von Guar gum mit breiiger Konsistenz aufgetragen und danach durch Borat-Ionen verfestigt. Dieses Wundauflagensystem hat sich im Prinzip bewährt, jedoch erfordert es in der Herstellung eine Reihe von Schritten, die der genauen Überwachung bedürfen, um Qualitätsschwankungen zu vermeiden. Weiterhin hat sich gezeigt, daß das mit Borat verfestigte Guar gum nur eine beschränkte Haftfestigkeit auf und im Polyurethanschaum aufweist und daher bei stärkerer mechanischer Belastung zur Abtrennung führt.

Aus der EP-A-0 190 814 ist eine Wundauflage mit geschlossenporigem Polyurethanschaum bekannt und einem darin verteilen Wasserabsorbens wie zum Beispiel Guargum.

Aus der US-A-5,065,752 sind hydrophile Schaumzubereitungen für medizinische Zwecke beschrieben, welche hohe Wassermengen aufnehmen sollen. Sie werden hergestellt, in dem gekappte Isocyanatpolyether Prepolymere und polymere hydrophile Mittel, die Wasser aufnehmen können und weitere Zusatzmittel miteinander geschäumt werden, wobei von vornherein eine klebende Komponente vorhanden ist, die auch später für die Klebrigkeit des Materials sorgt. Bei der Herstellung dieser Produkte können kleinen Mengen von Additiven zugesetzt werden, wie Methylcellulose, Guar gum, Pectin, Karaya gum, Chitosan, Agar, Akazienpulver, Carrageen, Gelatine und Kombinationen hiervon. Die Menge darf jedoch keinefalls so groß sein, daß dadurch die Wasseraufnahmekapazität des fertigen Schaums unter das 3-fache des Gewichtes sinkt; vgl. Spalte 7, Zeilen 41 bis 50. Vorzugsweise sollen die fertige Schäume mindestens das 50-fache ihres Gewichtes an Wasser aufnehmen können; vgl. Spalte 6, Zeile 50 bis Spalte 7, Zeile 2.

Die Erfindung hat sich somit die Aufgabe gestellt, das Wundauflagensystem gemäß WO 92/03172 weiter zu verbessern und seine Herstellung so zu vereinfachen, daß die Herstellung auch in großen Mengen zuverlässig und reproduzierbar erfolgen kann. Dabei ist jedoch darauf zu achten, daß die Wasseraufnahmekapazität des fertigen Produktes unter dem 3-fachen des Ausgangsgewichtes liegt. Vorzugsweise soll die Wasseraufnahmekapazität sogar nur beim 1- bis 2-fachen des Ausgangsgewichtes liegen. Die Wundverbände, Wundauflagen und Trägermatrixe gemäß der vorliegenden Erfindung sollen somit keinesfalls ein Austrocknen der Wunden bewirken, sondern in ähnlicher Weise wie natürliche Wundverschlüsse ein Gleichgewicht herstellen zwischen Feuchtigkeitsabgabe und Feuchtigkeitsaufnahme. Sobald ein Wundverband eine hohe Wasseraufnahmekapazität hat, führt dies zum Austrocknen der Wunde. Sofern der Wundverband keine ausreichende Wasserdampfdurchlässigkeit aufweist, führt dies zur Ansammlung von Flüssigkeit, die sich unter anderem durch sehr unangenehmen Geruch bemerkbar macht.

Diese Aufgabe kann überraschend einfach gelöst werden dadurch, daß das Guar gum sowie die Tenside und/oder weiteren Zusatzstoffe in situ mit eingeschäumt werden bei der Umsetzung von Wasser mit hydrophilen Polyurethan-Prepolymeren auf Basis von Toluoldiisocyanat (TDI) und/oder Methylendiphenyldiisocyanat (MDI), wobei die Menge an Guar gum 0,1 bis 15 % beträgt und so hoch gewählt wird, daß die Wasseraufnahmekapazität des fertigen Produktes unter dem 3-fachen des Ausgangsgewichtes liegt.

Die Herstellung dieser Wundverbände, Wundauflagen und Trägermatrices erfolgt somit dadurch, daß der Polyurethanschaum hergestellt wird aus mit Wasser schäumbaren hydrophilen Polyurethan-Prepolymeren auf Basis von Toluoldiisocyanat (TDI) und/oder Methylendiphenyldiisocyanat (MDI) in Gegenwart von ausreichenden Mengen an gelöstem Guar gum sowie Tensiden und/ oder weiteren Zusatzstoffen, so daß der Gehalt an Guar gum 0,1 bis 15 % beträgt, und die Wasseraufnahmekapazität des fertigen Produktes unter dem 3-fachen, vorzugsweise nur dem 1- bis 2-fachen des Ausgangsgewichtes liegt.

Als Zusatzstoffe kommen insbesondere in Frage Kollagen, Kollagenderivate, Cellulose, Cellulosederivate, Agar oder andere gelbildende Polysaccharide und Gemische derselben. Die Mengen liegen meistens bis zu 5 % in der wäßrigen Lösung. Weiterhin kommen in Frage die Wundheilung fördernde Peptide, bakterizide, antimykotische Substanzen und Gemische derselben. Schließlich kommen in Frage Enzyme, biologisch aktive Peptide wie zum Beispiel Oligopeptide des Kollagens oder des Fibronectins, autolog gezüchtete Zellverbände oder lebende Hautzellen. Diese Stoffe und Zellen werden vorzugsweise an die Oberfläche der Wundauflage mittels inerter Trägermaterialien wie zum Beispiel Perlcellulose bzw. Sepharose gebunden. In einem separaten Schritt werden zunächst an die Perlcellulose bzw. Sepharose die genannten Stoffe bzw. Zellen in bekannter Weise immobilisiert und dann an die Oberfläche der Wundauflage fest gebunden. Die Bindung der beladenen Perlcellulose- bzw. Sepharosemikrokugeln erfolgt vorzugsweise über das für die Herstellung des Schaumes bereits verwendete Polyurethan-Prepolymere. Dieses Prepolymere wird zum Beispiel punktförmig auf die Oberfläche der fertigen Wundauflage aufgetragen, und unmittelbar danach die beladenen Perlcellulose- bzw. Sepharosemikrokugeln, die an die Oberfläche des frisch aufgetragenen Prepolymeren anpolymerisiert werden. Die halbkugelförmig nach außen zeigenden Mikrokugeln tragen die an sie gebundene biologische Aktivität, die im Wundgebiet ungehindert zur Wirkung kommen kann.

Die Polyurethan-Prepolymere auf Basis von Toluoldiisocyanat (TDI) und/oder Methylendiphenyldiisocyanat (MDI) sind bekannt und werden beispielsweise von den Firmen Grace & Co. sowie Rexolin Chemicals AB unter der Bezeichnung Hypol^{R} hergestellt und vertrieben. Diese Prepolymere werden im allgemeinen mit Wasser etwa im Verhältnis 1:1 vermischt, wobei durch Art und Menge der Zusatzstoffe - insbesondere Detergenzien -Porengroße, Porenform und mechanische Eigenschaften des Schaumes gesteuert werden können. Die so erhaltenen Polyurethanschäume können vor allem ohne toxische Katalysatoren und sonstige unerwünschte Beimengen hergestellt werden, so daß sie prinzipiell gut geeignet sind, auch als Verbandstoffe eingesetzt zu werden. Erfindungsgemäß werden diese Schäume erstmals hergestellt in Gegenwart von gelöstem Guar gum sowie gegebenenfalls Tensiden und/oder weiteren Zusatzstoffen. Guar gum ist ein Peptidomannan, welches mit Wasser viskose, wäßrige Lösungen bildet, die bisher nur durch Zugabe einer Boratlösung in ein mechanisch stabiles Hydrogel überführt werden konnten. Erfindungsgemäß kann auf den Zusatz von Borat verzichtet werden. Die erfindungsgemäßen Produkte bestehen aus einem miteinander verwobenen und vernetzten Schaum aus Polyurethan und Guar gum. Der so erhaltene Schaum hat gegenüber Schäumen ohne Guar gum ein höheres Wasserrückhaltevermögen. Das gleiche gilt für Guar gum, welches mit Borat zum Hydrogel gefällt worden ist. Die erfindungsgemäßen Produkte sind somit in der Lage, bezogen auf ihr Eigengewicht relativ viel Wasser zu binden und dabei dennoch eine ausreichende mechanische Stabilität aufzuweisen. Im Gegensatz zu dem Wundauflagensystem gemäß WO 92/03172 ist es auch nicht möglich, das Hydrogel aus Guar gum mechanisch von dem Schaum zu trennen, so daß die mechanische Beanspruchbarkeit verbessert ist.

Das hohe Wasserrückhaltevermögen führt dazu, daß auch die fertigen Produkte im allgemeinen noch hohe Wassermengen enthalten. Die fertigen Produkte haben somit nur eine geringe Wasseraufnahmekapazität, die unter dem 3-fachen, vorzugsweise unter dem 1- bis 2-fachen des Ausgangsgewichtes liegt.

Der Gehalt an Guar gum ist in weiten Grenzen variierbar. Zwischen 0,1 und 15%, vorzugsweise 1 bis 5 % Guar gum, bezogen auf das Prepolymere, ergibt hervorragende Produkte. Gegenüber dem Wundauflagensystem gemäß WO 92/03172 fühlen sich die erfindungsgemäßen Produkte weicher und zarter an, obwohl sie bei mechanischer Beanspruchung stabiler sind als die Produkte nach dem Stand der Technik.

Durch bioverträgliche Detergenzien können Porengröße, Porenform, Wandstärke und sonstige Eigenschaften der fertigen, Guar gum enthaltenden Polyurethanschäume variiert werden. Mengen von 1 bis 3 % haben sich besonders bewährt. Auch die weiteren Zusatzstoffe haben Einfluß auf die endgültige Erscheinungsform des Schaumes, jedoch läßt sich dieses leicht regulieren und gut steuern.

Ein wesentlicher überraschender Vorteil der erfindungsgemäßen Produkte ist die Sterilisierbarkeit mit Gammastrahlen im feuchten Zustand ohne Verlust der Geleigenschaften. Bei der Sterilisierung mit Gammastrahlen von Guar gum, welches mit Borat zum Hydrogel gefällt worden ist, erfolgt eine stets unerwünschte Verflüssigung des Hydrogels.

Gewünschtenfalls können die erfindungsgemäßen Produkte in feuchtem, trockenem oder angetrocknetem Zustand gelagert und angewendet werden. Durch eine gegebenenfalls vorhandene Folie kann erreicht werden, daß die Oberfläche wasserundurchlässig ist, aber gas- und wasserdampfdurchlässig bleibt.

Sofern als Zusatzstoffe Kollagen, Kollagenderivate, Cellulose, Cellulosederivate, Agar oder andere gelbildende Polysaccharide und Gemische derselben verwendet werden, können weitere Verbesserungen erzielt werden. Insbesondere bei Zusatz von Kollagen und Kollagenderivaten erhält man noch weichere Produkte bei gleicher mechanischer Festigkeit.

Sofern als Zusatzstoffe die Wundheilung fördernde Peptide, bakterizide, antimykotische Substanzen und Gemische derselben verwendet werden, sind diese in der Lage, auch aus dem Inneren des erfindungsgemäß hergestellten Schaumes herauszudiffundieren und wirksam zu werden. Werden als Zusatzstoffe Enzyme, biologisch aktive Peptide, autolog gezüchtete Zellverbände oder lebende Hautzellen verwendet, so werden diese Zusatzstoffe vorzugsweise an die Oberfläche der Wundauflage über inerte Trägermaterialien mittels des zur Herstellung des Wundauflagenschaumes benutzten Prepolymeren fest angebunden.

Die erfindungsgemäßen Wundverbände, Wundauflagen und Trägermatrices können in üblicher Weise auf dem Körper aufgebracht werden, beispielsweise mit Hilfe eines Mullverbandes, Klebestreifen oder selbstklebenden Flächen auf Teilen der der Haut zugewandten Seite des Materials.

Prinzipiell ist es aber auch möglich gemäß US-A-5,065,752 schon ein klebende Komponente beizufügen, so daß selbstklebende Schäume entstehen.

In den nachfolgenden Beispielen ist die Herstellung einer erfindungsgemäßen Wundauflage näher erläutert.

Zunächst wird eine etwa 2%-ige Lösung eines nicht ionischen Detergens (Pluriol^{R} PE 6800/BASF) hergestellt. In 10 l dieser Lösung werden 760 g Guar gum-Pulver so eingerührt, daß ein homogenes Hydrogel entsteht. Dieses Hydrogel wird mit einem handelsüblichen Polyurethan-Prepolymer auf Basis von Toluoldiisocyanat (TDI) in einem Niederdruckmischkopf vermischt und in einer Form ausgeschäumt. Der so entstehende Schaumblock aus Guar gum in Polyurethan wird in ca. 4 mm dicke Wundauflagen zerschnitten.

Dieser Schaum kann in dieser Form oder in teilweise oder ganz getrockneter Form zur Anwendung kommen. Er ist mechanisch stabil, fühlt sich aber sehr weich und zart an. Er hat im allgemeinen eine Wasseraufnahmekapazität unter dem 3-fachen des Ausgangsgewichtes und ein sehr gutes Wasserhaltevermögen. Sofern die Wasseraufnahmekapazität des stärker getrockneten Materials mehr als das 3-fache beträgt, ist darauf zu achten, daß das fertige Produkt keinesfalls so stark getrocknet wird. Durch den Gehalt an Guar gum ist das Wasserhaltevermögen so hoch, daß keine ungewollte, nachträgliche Austrocknung stattfindet. Weiterhin ist zu beachten, daß bei Kompressionsverbänden die Wasseraufnahmekapazität der Wundauflage um das 1- bis 2-fache herabgesetzt wird. Die Wundauflage unter einem Kompressionsverband hat somit in der Praxis eine geringere Wasseraufnahmekapazität als der nicht kompressierte Wundverband. Eine geringfügige Übertrocknung und damit unerwünschte Erhöhung der Wasseraufnahmekapazität des Wundverbandes führt somit bei Kompressionsverbänden noch nicht zu der unerwünschten Austrocknung der Wunde, sowie zum unötigen Verbrauch von körpereigenen, für die Wundheilung essentiellen Stoffen aus dem Wundbereich. Angestrebt wird somit im Wundgebiet ein Gleichgewicht zwischen dem Wundexudat (körpereigene Flüssigkeit) und der in der Wundauflage befindlichen Feuchtigkeit, und daß über einen längeren Zeitraum von einigen Tagen.

Eine bevorzugte Rezeptur hat somit folgende Zusammensetzung: Es wird eine wäßrige Lösung hergestellt aus 1,8 % Guar gum, 2,5 % Pluronic^{R} 6800 (ein Tensid der BASF) und 0,1 % Kollagen. 1,5 Teile dieser Lösung werden mit einem Teil Hypol^{R} in einen Mischkopf geleitet, hier intensiv vermischt und in eine Form zur Schaumstoffbildung geleitet. Die Konzentration an Guar gum wird meist im Bereich zwischen 1 und 5 % gewählt. Niedrigere Konzentration als 0,1 % führen zu Produkten mit zu hoher Wasseraufnahmekapazität. Konzentrationen von über 5 % sind im allgemeinen wegen der hohen Viskosität schwerer handzuhaben. Die Viskosität und die Eigenschaften der Produkte hängen aber auch von weiteren Komponenten wie Kollagen ab, so daß es möglich ist, die gewünschten Eigenschaften des fertigen Wundverbandes auch den jeweiligen speziellen Anforderungen anzupassen. Sofern weitere für die Wundheilung wichtige Stoffe zugesetzt werden, können diese gelöst oder suspendiert zugesetzt werden.

Die Trocknung des fertigen Produktes erfolgt im allgemeinen nur teilweise, so daß noch eine erhebliche Menge von Wasser im Produkt bleibt. Feuchtigkeitsgehalte zwischen 20 und 60 % sorgen im allgemeinen dafür, daß auch trockene Wunden von vornherein zu einem ausreichend feuchten Wundmilieu führen.

Die Porigkeit des Schaums ist im allgemeinen gleichmäßig und überwiegend offen. Durch den Zusatz von wäßriger Kollagenlösung erhält man einen Schaum, der bei gleichen mechanischen Eigenschaften sich noch weicher und zarter anfühlt.

Bei Wiederholung des Versuches unter Zusatz der antimykotischen Substanz Clotrimazol entsteht ein Schaum, der auch in Fingerhutform zur Anwendung kommen kann und - auf Fingerspitzen oder Zehenspitzen gesteckt - zur Behandlung von Nagelmykosen geeignet ist. Durch die Einwirkung des schlecht löslichen Clotrimazols über 24 Stunden und länger kann der Wirkstoff in die tieferen Bereiche des Nagelbettes eindiffundieren und seine Wirkung entfalten.

## Patentansprüche

1. Wundverband, Wundauflage oder Trägermatrix, bestehend aus einem bioverträglichen, offenporigen Polyurethanschaum und einem eingelagerten Hydrogel aus Guar gum sowie Tensiden und/oder weiteren Zusatzstoffen, wobei das Guar gum sowie die Tenside und/oder weiteren Zusatzstoffe in situ miteingeschäumt werden bei der Umsetzung von Wasser mit hydrophilen Polyurethan-Prepolymeren auf Basis von Toluoldiisocyanat und/oder Methylendiphenyldiisocyanat, dadurch gekennzeichnet, daß der Gehalt an Guar gum 0,1 bis 15 % beträgt, und die Wasseraufnahmekapazität des fertigen Produktes unter dem 3-fachen des Ausgangsgewichts liegt.

2. Wundverband, Wundauflage oder Trägermatrix gemäß Anspruch 1, dadurch gekennzeichnet, daß die weiteren Zusatzstoffe Kollagen, Kollagenderivate, Cellulose, Cellulosederivate, Agar oder andere gelbildende Polysaccharide und Gemische derselben sind.

3. Wundverband, Wundauflage oder Trägermatrix gemäß Anspruch 1 oder 2, dadurch gekennzeichnet, daß die Zusatzstoffe die Wundheilung fördernde Peptide, bakterizide, antimykotische Substanzen und Gemische derselben sind.

4. Wundverband, Wundauflage oder Trägermatrix gemäß einem der Ansprüche 1 bis 3, dadurch gekennzeichnet, daß die Zusatzstoffe Enzyme, autolog gezüchtete Zellverbände oder lebende Hautzellen sind.

5. Wundverband, Wundauflage oder Trägermatrix gemäß einem der Ansprüche 1 bis 4, dadurch gekennzeichnet, daß Enzyme, biologisch aktive Peptide, autolog gezüchtete Zellverbände oder lebende Hautzellen, gebunden an inerte Trägerpartikel, über diese nachträglich an die Oberfläche fest gebunden sind.

6. Wundverband, Wundauflage oder Trägermatrix gemäß einem der Ansprüche 1 bis 5, dadurch gekennzeichnet, daß die der Wunde abgewandte Seite abgedeckt ist mit einer wasserundurchlässigen, aber gas- und wasserdampfdurchlässigen Schicht.

7. Verfahren zur Herstellung von Wundverbänden, Wundauflagen oder Trägermatrices, bestehend aus einem bioverträglichen, offenporigen Polyurethanschaum und einem eingelagerten Hydrogel aus Guar gum, wobei der Polyurethanschaum hergestellt wird aus mit Wasser schäumbaren hydrophilen Polyurethan-Prepolymeren auf Basis von Toluoldiisocyanat und/oder Methylendiphenyldiisocyanat in Gegenwart von gelöstem Guar gum sowie Tensiden und/oder weiteren Zusatzstoffen, dadurch gekennzeichnet, daß die Menge an Guar gum 0,1 bis 15 % beträgt und so hoch gewählt wird, daß die Wasseraufnahmekapazität des fertigen Produktes unter dem 3-fachen des Ausgangsgewichts liegt.

8. Verfahren gemäß Anspruch 7, dadurch gekennzeichnet, daß als weitere Zusatzstoffe verwendet werden Kollagen, Kollagenderivate, Cellulose, Cellulosederivate, Agar oder andere gelbildende Polysaccharide und Gemische derselben.

9. Verfahren gemäß Anspruch 7 oder 8, dadurch gekennzeichnet, daß als Zusatzstoffe verwendet werden die Wundheilung fördernde Peptide, bakterizide, antimykotische Substanzen und Gemische derselben.

10. Verfahren gemäß einem der Ansprüche 7 bis 9, dadurch gekennzeichnet, daß als Zusatzstoffe verwendet werden Enzyme, autolog gezüchtete Zellverbände oder lebende Hautzellen.

11. Verfahren gemäß einem der Ansprüche 7 bis 9, dadurch gekennzeichnet, daß Enzyme, biologisch aktive Peptide, autolog gezüchtete Zellverbände oder lebende Hautzellen, gebunden an inerte Trägerpartikel, über diese nachträglich an die Oberfläche fest gebunden werden.

## Claims

1. A dressing, wound pad or carrier matrix, consisting of a biocompatible open-pore polyurethane foam having a hydrogel of guar gum embedded therein, and surfactants and/or other additives, wherein said guar gum and the surfactants and/or other additives are incorporated in the foam in situ in a reaction of water with hydrophilic polyurethane prepolymers based on toluenediisocyanate and/or methylenediphenyldiisocyanate, characterized in that the guar gum content is from 0.1 to 15%, and the water absorption capacity of the finished product is less than three times its initial weight.

2. The dressing, wound pad or carrier matrix according to claim 1, characterized in that said other additives are collagen, collagen derivatives, cellulose, cellulose derivatives, agar or other gel-forming polysaccharides, or mixtures thereof.

3. The dressing, wound pad or carrier matrix according to claim 1 or 2, characterized in that said additives are wound healing promoting peptides, bactericidal or antimycotic substances, or mixtures thereof.

4. The dressing, wound pad or carrier matrix according to any of claims 1 to 3, characterized in that said additives are enzymes, autologously grown cell associations or living skin cells.

5. The dressing, wound pad or carrier matrix according to any of claims 1 to 4, characterized in that enzymes, biologically active peptides, autologously grown cell associations or living skin cells are bound to inert carrier particles through which they are firmly bound to the surface afterwards.

6. The dressing, wound pad or carrier matrix according to any of claims 1 to 5, characterized in that the side facing away from the wound is covered by a water-impermeable layer which is, however, permeable to gases and water vapor.

7. A method for the preparation of dressings, wound pads or carrier matrices consisting of a biocompatible open-pore polyurethane foam having a hydrogel of guar gum embedded therein, wherein said polyurethane foam is prepared from water-foamable hydrophilic polyurethane prepolymers based on toluenediisocyanate and/or methylenediphenyldiisocyante in the presence of dissolved guar gum and surfactants and/or other additives, characterized in that the amount of guar gum is from 0.1 to 15% and is chosen such that the water absorption capacity of the finished product is less than three times its initial weight.

8. The method according to claim 7, characterized in that collagen, collagen derivatives, cellulose, cellulose derivatives, agar or other gel-forming polysaccharides, or mixtures thereof, are used as said other additives.

9. The method according to claim 7 or 8, characterized in that wound healing promoting peptides, bactericidal or antimycotic substances, or mixtures thereof are used as said additives.

10. The method according to any of claims 7 to 9, characterized in that enzymes, autologously grown cell associations or living skin cells are used as said additives.

11. The method according to any of claims 7 to 9, characterized in that enzymes, biologically active peptides, autologously grown cell associations or living skin cells are bound to inert carrier particles through which they are firmly bound to the surface afterwards.

## Revendications

1. Pansement, couverture pour plaie ou matrice support, constitué d'une mousse de polyuréthanne à alvéoles ouverts, biocompatible, et d'un hydrogel incorporé, constitué de gomme de guar, ainsi que de tensioactifs et/ou d'autres additifs, la gomme de guar et les tensioactifs et/ou les autres additifs étant expansés les uns avec les autres in situ lors de la réaction d'eau avec des prépolymères de polyuréthanne hydrophiles à base de diisocyanate de toluène et/ou de diphényldiisocyanate de méthylène, caractérisé en ce que la teneur en gomme de guar est de 0,1 à 15 %, et que le pouvoir d'absorption de l'eau que présente le produit fini est inférieur à trois fois le poids initial.

2. Pansement, couverture pour plaie ou matrice support selon la revendication 1, caractérisé en ce que les autres additifs sont le collagène, les dérivés du collagène, la cellulose, les dérivés de la cellulose, la gélose ou d'autres polysaccharides gélifiables, et leurs mélanges.

3. Pansement, couverture pour plaie ou matrice support selon la revendication 1 ou 2, caractérisé en ce que les additifs sont des peptides favorisant la cicatrisation, des substances bactéricides antimycotiques et leurs mélanges.

4. Pansement, couverture pour plaie ou matrice support selon l'une des revendications 1 à 3, caractérisé en ce que les additifs sont des enzymes, des groupements cellulaires cultivés en culture autologue ou des cellules cutanées vivantes.

5. Pansement, couverture pour plaie ou matrice support selon l'une des revendications 1 à 4, caractérisé en ce que des enzymes, des peptides biologiquement actifs, des groupements cellulaires cultivés en culture autologue ou des cellules cutanées vivantes, fixés à des particules supports inertes, sont ensuite, par l'intermédiaire de ces dernières, fixés à demeure sur la surface.

6. Pansement, couverture pour plaie ou matrice support selon l'une des revendications 1 à 5, caractérisé en ce que la face opposée à la plaie est recouverte d'une couche imperméable à l'eau, mais perméable aux gaz et à la vapeur d'eau.

7. Procédé de fabrication de pansements, de couvertures pour plaie ou de matrices supports, constitués d'une mousse de polyuréthanne à alvéoles ouverts, biocompatibles, et d'un hydrogel incorporé, constitué de gomme de guar, où la mousse de polyuréthanne est fabriquée à partir de prépolymères de polyuréthanne hydrophiles, pouvant mousser à l'eau, à base de diisocyanate de toluène et/ou de diphényldiisocyanate de méthylène, en présence de gomme de guar dissoute et de tensioactifs, et/ou d'autres additifs, caractérisé en ce que la quantité de gomme de guar est de 0,1 à 15 % et est choisie à une valeur suffisamment grande pour que le pouvoir d'absorption de l'eau que présente le produit fini soit inférieur au triple du poids initial.

8. Procédé selon la revendication 7, caractérisé en ce qu'on utilise en tant qu'autres additifs le collagène, les dérivés du collagène, la cellulose, les dérivés de la cellulose, la gélose ou d'autres polysaccharides gélifiables, et leurs mélanges.

9. Procédé selon la revendication 7 ou 8, caractérisé en ce qu'on utilise en tant qu'additifs des peptides favorisant la cicatrisation, des substances bactéricides antimycotiques, et leurs mélanges.

10. Procédé selon l'une des revendications 7 à 9, caractérisé en ce qu'on utilise en tant qu'additifs des enzymes, des groupements cellulaires cultivés en culture autologue, ou des cellules cutanées vivantes.

11. Procédé selon l'une des revendications 7 à 9, caractérisé en ce que des enzymes, des peptides biologiquement actifs, des groupements cellulaires cultivés en culture autologue ou des cellules cutanées vivantes, fixés à des particules supports inertes, sont, par l'intermédiaire de ces dernières, fermement fixées après-coup à la surface.
